# EUROPÄISCHE PATENTANMELDUNG

(11) **EP 0 567 855 A1**
(43) Veröffentlichungstag der Anmeldung: **03.11.1993**
(21) Anmeldenummer: 93106132.9
(22) Anmeldetag: 15.04.1993
(51) Int. Cl.: C07C 37/84, C07C 39/16

(54) **Verfahren zur Herstellung von hochreinem Bisphenol-A durch fraktionierte Schmelzkristallisation**

(30) Priorität: 28.04.1992 DE 4213872
(71) Anmelder: BAYER AG, 51368 Leverkusen (DE)
(72) Erfinder: Malamet, Georg, Dr., W-4150 Krefeld (DE); Wirges, Hans-Peter, Dr., W-4150 Krefeld (DE); Wulff, Claus, Dr., W-4150 Krefeld (DE); Eitel, Alfred, Dr., W-4047 Dormagen (DE)

(57) **Zusammenfassung**

Die Erfindung betrifft ein spezielles Verfahren zur Herstellung von hochreinem Bisphenol-A aus Aceton und Phenol in Gegenwart saurer Katalysatoren, durch fraktionierende Schmelzkristallisation.

## Beschreibung

Die Erfindung betrifft ein spezielles Verfahren zur Herstellung von hochreinem Bisphenol-A aus Aceton und Phenol in Gegenwart saurer Katalysatoren.

Verfahren zur Herstellung von hochreinem Bisphenol-A aus Phenol und Aceton in Gegenwart saurer Katalysatoren sind bekannt. Beispielsweise kann das überschüssige Phenol abdestilliert und das zurückbleibende Rohbisphenol durch Umkristallisation aus organischen Lösemitteln wie Toluol gereinigt werden. Man kann auch aus dem Reaktionsgemisch das Bisphenol A-Phenoladdukt auskristallisieren, das darin enthaltene Phenol destillativ entfernen und das zurückbleibende Bisphenol, wie vorbeschrieben, umkristallisieren. Diese Verfahren haben den Nachteil, daß Fremdlösemittel verwendet und aufgearbeitet werden müssen.

Weiterhin ist bekannt, hochreines Bisphenol-A dadurch herzustellen, daß man aus dem Reaktionsgemisch den größten Teil des Phenols durch Destillation entfernt und das so erhaltene Rohbisphenol nahezu phenolfrei, d.h. mit weniger als 10 %, bevorzugt weniger als 3 % Phenolanteil durch fraktionierte Schmelzkristallisation in einem dynamischen Fallfilmkristallisator, wie er beispielsweise bei Rittner und Steiner in Chem.-Ing.-Techn. 57 (1985), 91 oder Wellinghoff und Wintermantel in Chem.-Ing.-Techn. 63 (1991), 881 beschrieben ist, reinigt. Dieses Verfahren hat den Nachteil, daß einmal sehr viel Phenol zunächst destillativ abgetrennt werden muß, was zu hohem Aufwand und erhöhter, längerer thermischer Belastung des Bisphenols-A und der Nebenprodukte führt. Zum anderen ist das erhaltene Rohbisphenol im allgemeinen stark verunreinigt, was eine nachgeschaltete Schmelzkristallisation über viele Stufen erfordert, bis die gewünschte hohe Reinheit erreicht wird.

Es wurde nun ein neues Verfahren zur Herstellung von hochreinem Bisphenol-A gefunden, das die genannten Nachteile nicht aufweist.

Gegenstand der Erfindung ist somit ein Verfahren zur Herstellung von hochreinem Bisphenol-A, das in üblicher Weise aus Phenol und Aceton in Gegenwart saurer Katalysatoren erzeugt wurde, aus der anfallenden Reaktionslösung, dadurch gekennzeichnet, daß die Reaktionslösung so weit abgekühlt wird, bis das Bisphenol in Form seines 1:1-Addukts mit Phenol auskristallisiert, dieses Addukt bei gleichzeitiger Rückführung der abgetrennten phenolischen Mutterlauge in das Verfahren abtrennt, dem abgetrennten Addukt soviel Phenol zugefügt wird, daß der Gehalt an Phenol mindestens 40 Gew.-% beträgt, dieses Gemisch durch fraktionierende Schmelzkristallisation hochreinigt und abschließend das so erhaltene Addukt durch Destillation in hochreines Bisphenol-A ( > 99 Gew.-%) und Phenol auftrennt, welches wieder in das Verfahren zurückgeführt wird.

Das erfindungsgemäße Verfahren liefert Bisphenol-A in hoher Reinheit von über 99,9 Gew.-%. Es weist folgende besonderen Vorteile auf: Der größte Teil des überschüssigen Phenols kann durch Filtration entfernt werden. Destilliert werden muß nur noch 1 Mol Phenol pro Mol Bisphenol. Weiterhin geht das erfindungsgemäße Verfahren von bereits stark vorgereinigtem Material von mindestens 99,0 Gew.-% p.p-Bisphenolgehalt aus, die Zahl der erforderlichen Stufen bei der Schmelzkristallisation wird stark verringert. Schließlich kann der Prozeß der Schmelzkristallisation bei dem thermolabilen Material bei den tieferen Schmelztemperaturen des Bisphenol-A Phenol-Addukts (100 bis 120°C gegenüber 160 bis 170°C bei phenolfreier Bisphenolkristallisation) ablaufen.

Zur Durchführung der fraktionierenden Schmelzkristallisation kann beispielsweise ein handelsüblicher Fallfilmkristallisator verwendet werden.

Gegebenenfalls kann das erfindungsgemäße Verfahren unter Inertgas (z.B. N₂, Edelgase usw.) durchgeführt werden.

### Beispiel

Durch Reaktion von Aceton mit überschüssigem Phenol wurde durch Katalyse an einem sauren Ionenaustauscher in bekannter Weise eine Lösung von Rohbisphenol (p.p-Bisphenol mit Nebenprodukten wie o.p-Bisphenol, Indanen, Chromanen und Trisphenolen) erzeugt. Diese Reaktionslösung wurde in einem Kühlungskristallisator auf 45°C abgekühlt, wobei das 1:1 Addukt von schon relativ reinem p.p-Bisphenol und Phenol auskristallisierte. Das auskristallisierte Addukt wurde abfiltriert und mit Phenol gewaschen, das gesammelte Filtrat wurde vor die Reaktion zurückgeführt.

Das abfiltrierte Addukt bestand aus 62 Gew.-% Bisphenol und 38 Gew.-% Phenol, wobei der Gehalt an p.p-Bisphenol bei 99,70 Gew.-% lag und folgende Nebenprodukte enthielt:

| | |
|---|---|
| o.p-Bisphenol | 1390 ppm |
| Indane | 40 ppm |
| Chromane | 300 ppm |
| Trisphenol I | 200 ppm |
| Trisphenol II | 800 ppm |
| Unbekannte | 270 ppm |

Diesem Addukt wurde soviel Phenol zugesetzt, daß ein Gemisch aus 44,2 Gew.-% Phenol und 55,8 Gew.-% Bisphenol entstand. Das Gemisch wurde in einem Sulzer-Fallfilmkristallisator (Rohrlänge 12 m, Rohrdurchmesser 70 mm) einer 2stufigen Schmelzkristallisation unterworfen. Diese fraktionierende Schmelzkristallisation wurde im Temperaturbereich von 100°C für das Kristallisieren, 100 bis 120°C für das Schwitzen und 120 bis 130°C für das Abschmelzen des hochgereinigten Addukts durchgeführt.

Abschließend wurde aus dem hochgereinigten Addukt das Phenol durch Destillation entfernt und in die Reaktion zurückgeführt. Das erhaltene hochreine Bisphenol wies eine Reinheit von 99,91 Gew.-% auf und die Nebenprodukte hatten sich wie folgt verringert:

| | |
|---|---|
| o.p-Bisphenol | 230 ppm |
| Indane | 0 ppm |
| Chromane | 140 ppm |
| Trisphenol I | 50 ppm |
| Trisphenol II | 330 ppm |
| Unbekannte | 150 ppm |

## Patentansprüche

1. Verfahren zur Herstellung von hochreinem Bisphenol-A, das in üblicher Weise aus Phenol und Aceton in Gegenwart saurer Katalysatoren erzeugt wurde, aus der anfallenden Reaktionslösung, dadurch gekennzeichnet, daß die Reaktionslösung so weit abgekühlt wird, bis das Bisphenol in Form seines 1:1-Addukts mit Phenol auskristallisiert, dieses Addukt bei gleichzeitiger Rückführung der abgetrennten phenolischen Mutterlauge in das Verfahren abtrennt, dem abgetrennten Addukt soviel Phenol zugefügt wird, daß der Gehalt an Phenol mindestens 40 Gew.-% beträgt, dieses Gemisch durch fraktionierende Schmelzkristallisation hochreinigt und abschließend das so erhaltene Addukt durch Destillation in hochreines Bisphenol-A und Phenol auftrennt, welches wieder in das Verfahren zurückgeführt wird.
